(19) Europäisches Patentamt
European Patent Office
Office européen des brevets

(11) EP 0 566 370 B1

# (12) EUROPEAN PATENT SPECIFICATION

(45) Date of publication and mention of the grant of the patent:
**29.01.1997 Bulletin 1997/05**

(21) Application number: **93302872.2**

(22) Date of filing: **14.04.1993**

(51) Int Cl.6: **C07C 69/16**, C07C 67/37,
C07C 51/54, C07C 53/12,
C07C 69/14, C07C 67/293,
C07C 29/151, C07C 31/04,
C07C 41/09, C07C 43/04

(54) **Integrated process for synthesizing oxygenated acetyl compounds from synthesis gas via dimethyl ether**

Integriertes Verfahren zum Herstellen von acetylierten Sauerstoffverbindungen aus Synthesegas über Dimethyläther

Procédé intégré de fabrication de composés oxygénés acétylés à partir du gaz de synthèse via l'éther diméthylique

(84) Designated Contracting States:
**BE DE FR GB IT NL**

(30) Priority: **15.04.1992 US 870126**
**20.10.1992 US 963771**

(43) Date of publication of application:
**20.10.1993 Bulletin 1993/42**

(73) Proprietor: **AIR PRODUCTS AND CHEMICALS, INC.**
**Allentown, PA 18195-1501 (US)**

(72) Inventors:
• **Studer, David William**
  **Wescosville, Pennsylvania 18106 (US)**
• **Waller, Francis Joseph**
  **Allentown, Pennsylvania 18103-9670 (US)**

(74) Representative: **Burford, Anthony Frederick**
**W.H. Beck, Greener & Co.**
**7 Stone Buildings**
**Lincoln's Inn**
**London WC2A 3SZ (GB)**

(56) References cited:
**EP-A- 0 077 116**          **GB-A- 1 538 782**

## Description

This invention relates to an integrated process for synthesizing ethylidene diacetate, acetic anhydride, methyl acetate, and acetic acid, and in particular the production of these oxygenated acetyl compounds from synthesis gas via the intermediate compound dimethyl ether.

Ethylidene diacetate (EDDA) is a valuable intermediate in the production of vinyl acetate (VAc), and considerable interest has been focused on developing improved processes for producing ethylidene diacetate. The commercial success of these improved processes, however, requires a market for the acetic acid (HOAc) which is a coproduct of vinyl acetate production. The acetic acid can be sold, esterified with methanol to methyl acetate (MeOAc), or alkylated with dimethyl ether (DME) to methyl acetate and methanol (MeOH). Methyl acetate (MeOAc) and acetic anhydride $(Ac_2O)$ are also important as intermediates for the production of other valuable products.

Representative processes for preparing EDDA include DE-A-2,610,035 which discloses a process for producing EDDA wherein the acetic acid obtained as a coproduct can be directly obtained by distillation processes and purified so that it can be used as such or reacted with methanol to form methyl acetate.

GB-A-1,538,782 describes a process for producing EDDA wherein dimethyl ether (DME) and/or methyl acetate, carbon monoxide and hydrogen are reacted in the presence of a catalyst system. The reaction preferably occurs in the presence of a Group VIII metal catalyst and a promoter such as an organo-phosphine and/or organo-nitrogen compound.

EP-A-0035860 discloses a process for producing EDDA and/or acetaldehyde wherein dimethyl ether or methyl acetate, carbon monoxide and hydrogen are reacted in the presence of a supported palladium catalyst and an halide.

An improved process is described in US-A-4,319,038 for preparing EDDA and acetic anhydride wherein methyl acetate and/or dimethyl ether, carbon monoxide and hydrogen are reacted in the presence of a quaternary nitrogen, and a manganese or rhenium compound.

EP-A-0077116 discloses a process for producing EDDA wherein dimethyl ether and/or methyl acetate, carbon monoxide and hydrogen are reacted in the presence of a catalyst system comprising a rhodium compound, a halogen component and a palladium co-catalyst.

EP-A-0058442 discloses a process for the coproduction of an alkylidene dicarboxylate and a carboxylic acid by the hydrogenation of a carboxylic acid anhydride in the presence of carbon monoxide and a homogeneous Group VIII metal catalyst together with a chloride, bromide, or iodide and a promoter comprising an organo oxygen, nitrogen, phosphorous, arsenic, or antimony compound having a lone pair of electrons.

US-A-4,323,697 discloses a process for preparing EDDA wherein methyl acetate and/or dimethyl ether, carbon monoxide and hydrogen are reacted in the presence of a molybdenum-nickel or tungsten-nickel co-catalyst in the presence of a promoter comprising an organo-phosphorous compound or an organo-nitrogen compound. When dimethyl ether is utilized, the reference teaches that a reactor having two reaction zones is preferred. In the first zone, DME is converted by carbonylation to methyl acetate and the second zone is devoted to conducting the EDDA-forming reaction.

US-A-4,429,150 discloses a process for producing EDDA wherein methyl acetate and/or dimethyl ether, carbon monoxide and hydrogen are reacted in the presence of a catalyst system comprising a Group VIII metal and a halogen-containing compound in the presence of a sulphur-containing polar solvent, e.g. sulpholane. The reference teaches that organo-phosphorous compounds improve selectivity and increase conversion to EDDA.

An integrated process for the production of synthesis gas is described in US-A-4,430,096 wherein one or more organic compounds are converted into hydrogen and carbon monoxide by partial oxidation in the presence of steam and/or carbon dioxide. The heat for the reaction is provided by direct heat exchange with products from the gasification of coal with oxygen and steam.

US-A-4,843,170 discloses a process for preparing vinyl acetate wherein dimethylacetal and acetic anhydride are converted to EDDA and methyl acetate in one of the steps.

The preparation of dimethyl ether from synthesis gas in a single stage liquid phase reactor containing solid methanol synthesis and methanol dehydration catalysts slurried in an inert oil is disclosed in EP-A-0324475 and in the article entitled "Single-step Synthesis of Dimethyl Ether in a Slurry Reactor" by J. J. Lewnard et al in <u>Chemical Engineering Science</u> Vol. 45, No. 8, pp. 2735-2741, 1990.

EDDA thus can be produced by several different process sequences according to the prior art. There is need for an improved integrated process for producing EDDA from synthesis gas with controlled coproduction of acetic acid, and in specific cases with minimum coproduction of acetic acid, while simultaneously maximizing carbon utilization in the synthesis gas feed. In addition, there is need for an improved method for producing vinyl acetate from EDDA with minimum coproduction of acetic acid. Further, the coproduction of the valuable compounds methyl acetate and acetic anhydride is desirable under certain market conditions. The invention described in the following specification and defined by the appended claims provides a new integrated process which fulfils these needs.

The invention is a process for the synthesis of oxygenated acetyl compounds from synthesis gas comprising hydrogen and carbon monoxide which comprises reacting the synthesis gas in a first liquid phase reactor in the presence of a methanol synthesis catalyst and a methanol dehydration catalyst suspended in an inert liquid at conditions sufficient to produce dimethyl ether and methanol. An intermediate product stream comprising dimethyl ether, methanol, water, and unreacted synthesis gas is withdrawn from the reactor and passed into a second liquid phase reactor in which the dimethyl ether, methanol, and unreacted synthesis gas are reacted with acetic acid in the presence of a catalyst system consisting essentially of a Group VIII metal, methyl iodide, lithium iodide and, optionally, lithium acetate at conditions sufficient to produce one or more oxygenated acetyl compounds selected from ethylidene diacetate, acetic acid, acetic anhydride, and methyl acetate. A liquid product mixture comprising one or more of these oxygenated acetyl compounds and a vapor stream comprising unreacted synthesis gas are withdrawn from the second liquid phase reactor.

Preferably, the synthesis gas is produced by the partial oxidation of a hydrocarbon feedstock, typically natural gas. In a most preferred embodiment of the invention, acetic acid and ethylidene diacetate are separated from the liquid product mixture, one portion of the acetic acid is recycled to the second liquid phase reactor, and optionally another portion is recycled to the partial oxidation reactor to generate additional synthesis gas. At least a portion of the unreacted synthesis gas from the second reactor also can be recycled to the partial oxidation reactor to enhance carbon recovery.

In a further embodiment, the ethylidene diacetate is passed into a pyrolysis reactor system which converts the ethylidene diacetate into an intermediate product comprising vinyl acetate and acetic acid, which is separated by distillation into vinyl acetate and acetic acid as final products. At least a portion of the acetic acid is recycled to the partial oxidation reactor to generate additional synthesis gas.

Acetic anhydride and methyl acetate also can be separated and recovered from the liquid product mixture as additional individual products. The net production of these compounds relative to EDDA and acetic acid can be increased by removing hydrogen from the unreacted synthesis gas prior to the second liquid phase reactor.

The present invention has several advantages over prior art methods for producing EDDA and vinyl acetate as well as the coproducts methyl acetate and acetic anhydride. First, the liquid phase dimethyl ether (LP DME) and liquid phase oxygenated acetyl (LP OA) reactors can be directly coupled wherein the feed requirements of the OA reactor closely match the typical product stream from the liquid phase DME reactor. Thus the removal of methanol and water from the DME reactor effluent is not required, but optionally may be carried out to optimize the amount of acetic acid produced. In addition, the DME and OA reactor systems can be integrated efficiently with the partial oxidation synthesis gas generation system by recycling selected amounts of acetic acid coproduct as well as unreacted synthesis gas to the partial oxidation reactor, which maximizes carbon utilization for the desired products. Further, the integrated reaction system can be operated flexibly to produce economically desirable amounts of the EDDA coproducts acetic acid, acetic anhydride, and methyl acetate.

The following is a description, by way of example only and with reference to the accompanying drawings, of a presently preferred process of the invention. In the drawings:

Fig. 1 is a block diagram of the integrated process of the present invention;
Fig. 2 is a general flow diagram of the liquid phase dimethyl ether reactor and separation system of the present invention; and
Fig. 3 is a general flow diagram of the liquid phase OA reactor and separation system of the present invention.

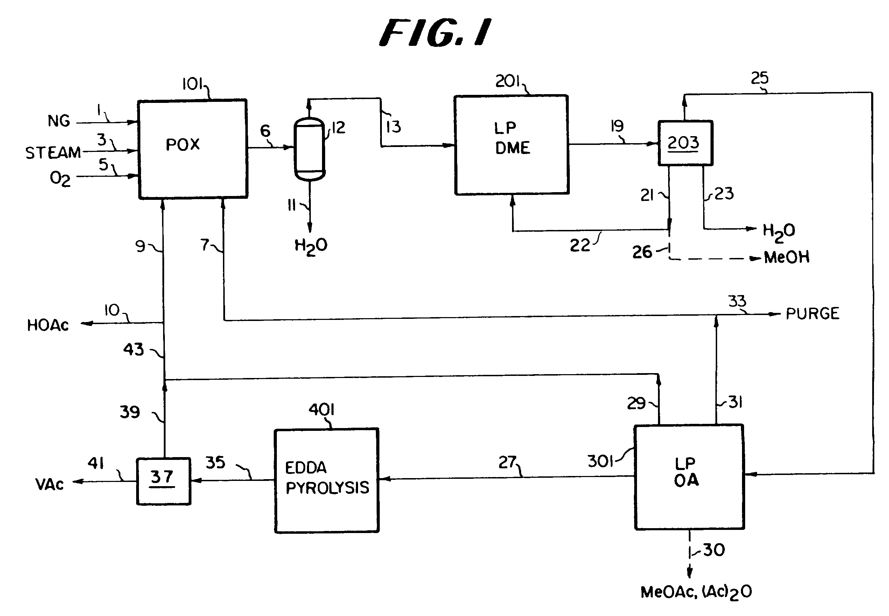

A process block flow diagram for the overall integrated process is shown in Figure 1. Hydrocarbon feedstock 1, preferably natural gas, is fed with steam 3 and oxygen 5 into partial oxidation (POX) reactor 101 to produce raw synthesis gas 6 containing typically 25 to 65 vol% hydrogen, 30 to 50 vol% carbon monoxide, 0.5 to 12 vol% carbon dioxide, 0 to 0.5 vol% methane, and 2 to 25 vol% water. The hydrocarbon feedstock alternately can be selected from methane, $C_2^+$ gaseous hydrocarbons, naphtha, gas oil, vacuum residuum, and various other combustible hydrocarbons including petroleum coke and coal. POX processes for synthesis gas generation are well known in the art and are offered commercially by Texaco and Shell, among others. Steam is both a reactant and a temperature moderant in the POX reactor. $CO_2$-rich unreacted synthesis gas recycle stream 7 typically containing 60-70 vol% $CO_2$ is introduced optionally into the POX reactor, wherein the $CO_2$ is both a reactant and a temperature moderant. In some cases, depending on the hydrocarbon feedstock used, the $CO_2$-rich recycle stream 7 may eliminate the need for steam 3. Coproduct acetic acid recycle stream 9 provides additional hydrocarbon feed to the POX unit and is converted into additional synthesis gas, thus reducing the hydrocarbon feedstock requirement. A portion 10 of acetic acid stream 9 optionally is withdrawn as an external product.

Condensable water 11 is removed in separator 12 and dry synthesis gas 13 passes into liquid phase dimethyl ether (DME) reactor system 201. Optionally, depending on the overall process carbon balance requirements, a portion of the hydrogen in feed 13 can be removed by pressure swing adsorption or cryogenic distillation. Product stream 19, comprising DME, methanol, unreacted synthesis gas (including $CO_2$), and water, passes to separation system 203 which yields methanol stream 21, water 23, and intermediate product stream 25 comprising DME and unreacted synthesis gas. Optionally, a portion 26 of methanol stream 21 is taken as an external product and another portion 22 is recycled to the LP DME reactor system. Alternately, separation system 203 is not used and stream 19 passes directly to LP OA reactor system 301. Alternatively, at least a portion of the carbon dioxide in stream 25 is removed by methods known in the art prior to the LP OA reactor system described below. Alternatively, some or all of the hydrogen in the unreacted synthesis gas from the LP DME reactor can be removed by condensing the DME and methanol and separating the resulting synthesis gas by known methods of pressure swing adsorption (preferably) or cryogenic distillation.

Oxygenated acetyl (OA) reactor system 301 comprises a liquid phase reactor in which DME, acetic acid, and synthesis gas react in the presence of one or more catalysts described below to yield EDDA and intermediates or coproducts including acetic acid, acetic anhydride, and methyl acetate. EDDA product 27, acetic acid 29, and unreacted synthesis gas 31 are separated from other coproducts which are recycled within reactor system 301. Optionally, a portion 30 of the coproducts acetic anhydride and methyl acetate can be withdrawn and separated into individual products. A small portion 33 of unreacted synthesis gas 31 is removed as purge, and the remainder 7 is recycled to POX reactor 101. EDDA product passes into EDDA pyrolysis system 401 where EDDA is thermally cracked to yield intermediate product 35 containing acetic acid (HOAc) and vinyl acetate (VAc), and this stream passes to separation system 37 which yields acetic acid 39 and vinyl acetate product 41. EDDA pyrolysis in system 401 and product separation in system 37 are known in the art, and any commercially available process is suitable for this purpose. A description of a commercial EDDA pyrolysis reaction and separation system is given for example in SRI Report No. 146, Process Economics Program Series, Stanford Research Institute, 1981.

Acetic acid streams 29 and 39 are combined into a total coproduct acetic acid stream 43, a portion 10 optionally is taken as a product, and the remainder 9 is recycled to POX reactor system 101 to generate additional synthesis gas. The amount of acetic acid product 10 will depend upon market and pricing conditions at a given plant location, and if desired the entire acetic acid stream 43 can be recycled to POX reactor system 101. Typically 0 to 50% of stream 43 is taken as acetic acid product 10.

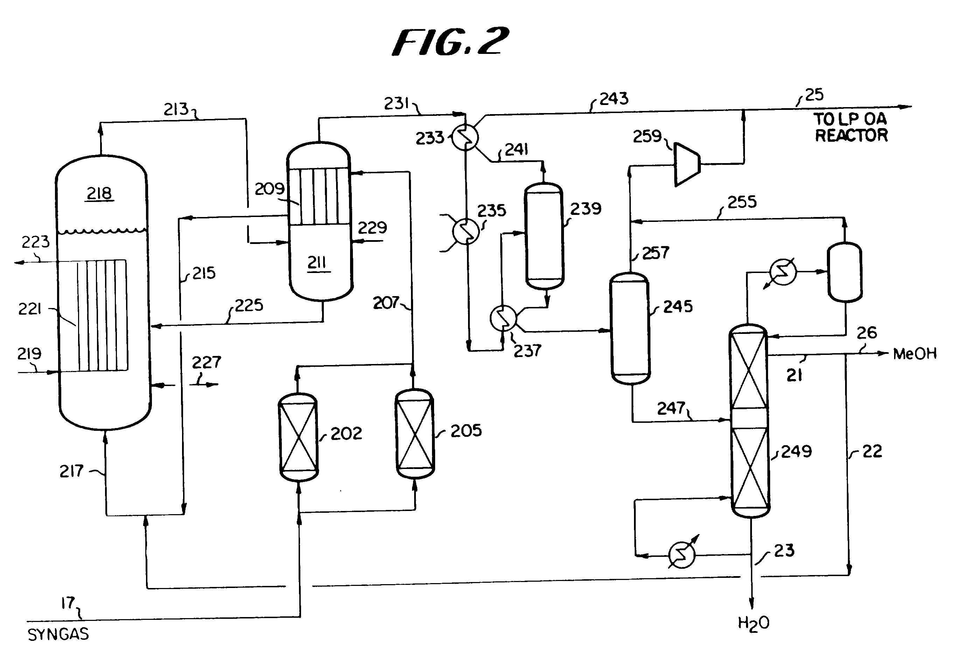

DME reactor system 201 is illustrated in more detail in Fig. 2. Feedstream 17 (equivalent to stream 13 of Fig. 1) is treated in alternating adsorbers 202 and 205 to remove metal carbonyl compounds and other contaminants which are detrimental to the DME synthesis catalysts. Clean synthesis gas 207 is heated to 300 to 430°F (150 to 220°C) in exchanger 209 in vessel 211 by indirect heat exchange with DME reactor effluent 213, heated synthesis gas 215 is optionally combined with methanol recycle 22, and combined feed 217 is introduced into liquid phase DME reactor 218. DME reactor 218 contains a methanol synthesis catalyst and a methanol dehydration catalyst, both in powdered form with an average particle size between 5 and 50 micrometers, suspended in an inert liquid. The methanol synthesis catalyst is selected from commercially-available copper/zinc-based catalysts, preferably a $Cu/ZnO/Al_2O_3$ catalyst such as the widely-used BASF S3-86. The methanol dehydration catalyst is selected from alumina, silica-alumina, zeolites such as ZSM-5, solid acids such as boric acid, solid acidic ion-exchange resins, and mixtures thereof. Typically a commercially-available alumina such as Catapal B gamma alumina may be used. The preferred alumina is prepared by heating boehmite (alumina monohydrate, $Al_2O_3 \cdot H_2O$) powder at a rate sufficient to increase the temperature of the alumina by about 100°C per hour to about 500°C, holding at this temperature for about 3 hours, and cooling the resulting heat-treated alumina to ambient temperature. When the methanol synthesis catalyst comprises copper and the methanol dehydration catalyst comprises alumina, it is preferred that the methanol synthesis catalyst is between 75 and 90% by weight of the total weight of methanol synthesis catalyst and methanol dehydration catalyst. The inert liquid for the catalyst slurry preferably comprises paraffinic or naphthenic hydrocarbons boiling in the range of 150 to 450°C. Alternatively alcohols, ethers, or polyethers with boiling points in this range can be utilized.

The synthesis gas reacts in the presence of the catalyst suspended in the inert liquid to form methanol and a significant portion of the methanol is dehydrated to form DME. Reactor effluent 213 contains typically 3-13 vol% DME, 1-5 vol% methanol, 40-75 vol% unreacted synthesis gas, and 0.2-1 vol% water. The synthesis and dehydration reactions are exothermic and the heat generated is removed by passing coolant 219 (preferably water) through exchanger 221 and withdrawing heated coolant 223 (preferably steam) therefrom. Reactor effluent 213 is cooled in exchanger 209 against feed 207 to condense and coalesce vaporized and entrained inert liquid, which accumulates in the lower section of vessel 211; the collected liquid 225 is returned to reactor 218. Spent catalyst slurry is withdrawn and fresh catalyst slurry is added to reactor 218 through line 227. Makeup inert liquid 229 is added to vessel 211 as needed. Reactor 218 is operated in the temperature and pressure ranges of 440 to 520°F (225 to 270°C) and 750-2000 psig (5-14 MPa) respectively. The reactor gas hourly space velocity (GHSV) is typically in the range of 3,000 to 15,000 std. liters/(kg cat·h).

DME-containing stream 231, which also contains methanol, unreacted synthesis gas, and water, is cooled in exchangers 233, 235, and 237 and passes to separator 239. Vapor 241 comprising unreacted synthesis gas and DME is warmed in exchanger 233 to yield stream 243. Liquid from separator 239 is partially vaporized in exchanger 237 and passes into separator 245, from which methanol-rich liquid stream 247 flows to distillation column 249. This column separates stream 247 into water waste bottoms stream 23, sidestream 21 containing 95-100 vol% methanol, and overhead stream 255 containing DME and unreacted synthesis gas. Stream 255 is combined with stream 257 which also contains DME and unreacted synthesis gas, the combined stream is compressed in compressor 259, and is combined with stream 243 to yield DME-synthesis gas stream 25 which provides the feed to OA reactor system 301 of Fig. 1. A portion 22 of methanol sidestream 21 is recycled to DME reactor 218, and the remainder 26 optionally is taken as a methanol product. Optionally, depending on the desired product slate of the overall process, all of methanol sidestream 21 can be taken as product or can be recycled totally to reactor 218 thereby increasing DME yield in stream 25.

Alternatively, as earlier described, stream 231 (identical to stream 19 of Fig. 1) containing DME, methanol, water, and unreacted synthesis gas is fed directly to OA reactor system 301 of Fig. 1. This alternate mode is possible when stream 231 contains less than 2 vol% water, preferably less than 1 vol% water.

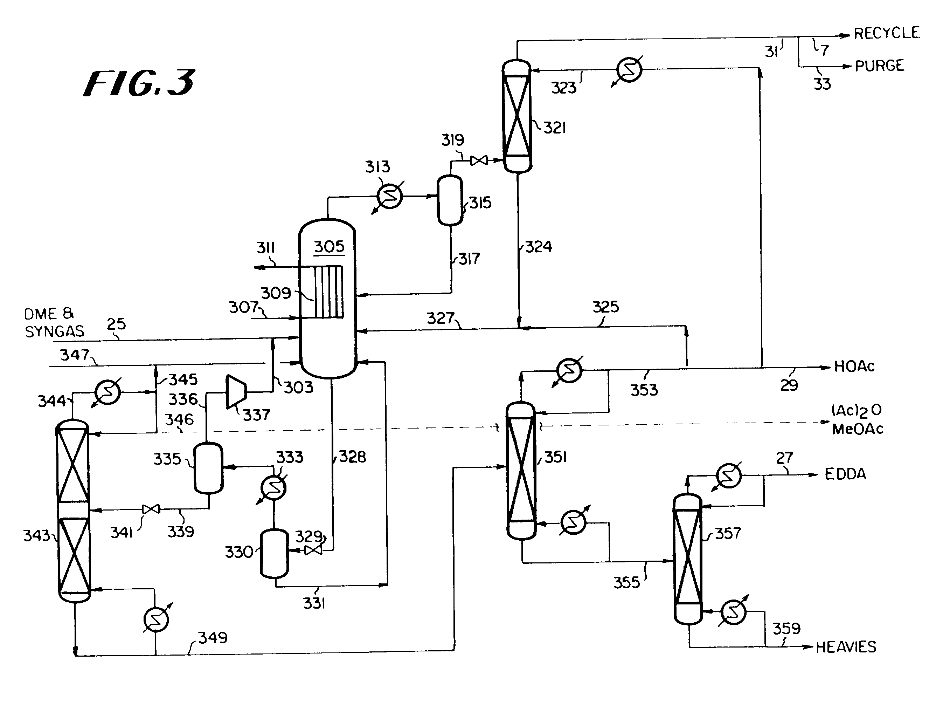

The OA reactor system is illustrated in more detail in Fig. 3. Stream 25 from the DME reactor system of Fig. 2, or alternatively stream 231 without water and methanol removal, is combined with recycle stream 303 and flows into OA liquid phase reactor 305. The liquid phase in the reactor comprises acetic acid, EDDA, and other reaction intermediates or coproducts comprising one or more of the components acetic anhydride, methyl acetate, and acetaldehyde. The major component is acetic acid which comprises 30 to 80 mol% of the total liquid in the reactor. The liquid contains a catalyst system, preferably soluble therein, which promotes the reaction of dimethyl ether, acetic acid, hydrogen, and carbon monoxide to form EDDA, acetic acid, and the other intermediates or coproducts identified earlier. Thus acetic acid is both a reactant and a product, and comprises the major liquid component in the liquid phase reactor. CO and hydrogen also react with one or more of the intermediates or coproducts identified above in a hydrocarbonylation reaction sequences which yield EDDA and acetic acid. While the exact reaction sequence is not fully understood, it is known in the present invention as later described that EDDA is a product and that acetic acid is a reactant as well as a coproduct.

The catalyst system is a new combination of catalysts which provides superior selectivity to EDDA and which can be operated under shorter reaction times than typically required in prior art processes for producing EDDA. The newly discovered catalyst system consists essentially of a Group VIII metal, methyl iodide, lithium iodide, and optionally lithium acetate, which in combination, provide superior results than achieved in prior art processes for producing EDDA which utilize a catalyst system containing less than the combination of these components.

The term hydrocarbonylation as used herein refers to the reaction of dimethyl ether, acetic acid, other intermediate components, hydrogen, and carbon monoxide to form EDDA, acetic acid, acetic anhydride, and methyl acetate under the described process conditions. Under certain conditions, especially at longer reactor residence times, acetaldehyde will be produced in moderate amounts. Hydrocarbonylation can be carried out in a batch mode or a continuous mode over a wide range of temperatures. While the optimum temperature for practicing the present invention will depend upon process stoichiometry, the particular catalyst system utilized, as well as the precise combination of reaction conditions, suitable hydrocarbonylation temperatures will range from 20°C up to 220°C. However, the most preferred hydrocarbonylation temperatures range from 120°C to 195°C. The hydrocarbonylation reaction can be carried out under a wide variety of pressures including pressures ranging from 100 psig (0.8 MPa) to 3000 psig (21 MPa). Preferred pressures range from 400 psig (3 MPa) to 2100 psig (15 MPa).

The catalyst system of the present invention utilizes a Group VIII metal selected from rhodium, platinum, palladium, iridium, ruthenium, cobalt and nickel with preferred Group VIII metals being rhodium and iridium. The Group VIII metal catalyst used in the catalyst system is present in a catalytically active amount and such catalytically effective amounts can be readily determined by those of ordinary skill in the art. The amount of Group VIII metal to be incorporated into the catalyst system typically ranges from 0.01 mol% to 10 mol% based on the DME present, preferably from 0.05 to 5 mol%. The most preferred Group VIII metal is rhodium.

Examples of suitable rhodium compounds to be incorporated into the catalyst system include rhodium oxide, rhodium (III) hydroxide, rhodium (III) chloride, rhodium (III) chloride trihydrate, rhodium (III) bromide, rhodium (III) iodide, rhodium (II) acetate, tetrarhodium dodecaacetyl, hexarhodium hexadecaacetyl, rhodium (I) diacetyl acetylacetonate, tris(pyridine)rhodium (III) chloride, chlorotris-(triphenylphosphine) rhodium and other organo-rhodium complexes. The preferred rhodium compound to be incorporated into the catalyst system is rhodium (III) chloride trihydrate which is commercially available in hydrated forms.

Examples of suitable palladium compounds to be incorporated into the catalyst system include palladium chloride, palladium chloride dihydrate, palladium bromide, palladium iodide, palladium oxide, palladium acetate, palladium butyrate and palladium acetylacetonate. Preferred palladium compounds include palladium chloride, palladium chloride dihydrate and palladium acetate.

In addition to the Group VIII metal, the catalyst system also contains methyl iodide and lithium iodide, in combination, which serve as promoters for driving the hydrocarbonylation reaction to completion. Applicants have discovered that unexpectedly superior conversion of dimethyl ether to EDDA occurs when lithium acetate is used in conjunction with both lithium iodide and methyl iodide. The amount of lithium iodide and methyl iodide used in conjunction with the desired Group VIII metal catalyst is not critical to practicing the present invention. The collective amount of the iodide components, (i.e., methyl iodide and lithium iodide) can be varied between wide limits.

Reaction time is a convenient control parameter in practicing the present invention and optimum reaction times can be determined based upon the enumerated reaction conditions, catalyst system, and catalyst concentration presented herein. Reaction times required to produce desired amounts of products will also depend upon the reaction temperature and pressure. At constant temperature, pressure, and catalyst concentration, shorter reaction times generally will result in the production of more methyl acetate, acetic anhydride, and acetic acid than EDDA, with very little acetaldehyde. At longer residence times, the production of acetic anhydride decreases significantly, while EDDA and acetic acid production increase significantly. Thus reactor residence time is a useful variable to control product distribution. The reaction is preferably run in the liquid phase containing a high proportion of acetic acid which, as earlier discussed, is a reactant in the present process as well as a coproduct produced therefrom. The liquid also will contain various amounts of the coproducts acetic anhydride, methyl acetate, and EDDA. A non-reactive dipolar solvent may be utilized in conjunction with these components.

The reactions which occur in reactor 305 are exothermic, and the heat generated is removed by passing cooling fluid 307, preferably water, through heat exchanger 309 disposed in reactor 305, and withdrawing heated fluid 311, preferably steam, therefrom. Vapor phase reaction products and other volatile components in the reactor overhead are cooled and partially condensed in cooler 313 and flow to separator 315. Condensate 317 is withdrawn therefrom and returned to reactor 305, and vapor 319 is passed into absorber 321. Cool acetic acid stream 323 passes downward through absorber 321 and absorbs any residual coproducts and volatile catalyst components, rich absorber solvent 324 is combined with acetic acid recycle 325, and combined stream 327 is returned to reactor 305. Absorber overhead stream 31 contains unreacted synthesis gas components and is rich in $CO_2$; a portion 7 thereof is recycled to POX reactor system 101 and the remaining portion 33 is removed as purge to prevent buildup of inert gas components such as argon.

Reactor liquid product stream 328 is flashed across valve 329 and flows into separator 330. Liquid stream 331, containing typically about 10% of the methyl acetate and acetic anhydride in reactor liquid effluent 328 and 15% of the acetic acid in stream 328, is returned to reactor 305. Stream 331 also contains some soluble catalyst components including lithium iodide, lithium acetate, and/or rhodium compounds. The vapor from separator 330 is partially condensed in cooler 333 and flows into separator 335 from which is withdrawn vapor stream 336 comprising DME, methyl iodide promoter, and unreacted synthesis gas components; this vapor stream is compressed by compressor 337 and compressed recycle stream 303 is combined with reactor feed 25.

Liquid 339 from separator 335, comprising EDDA, acetic acid, acetic anhydride, methyl acetate, and the catalyst component methyl iodide, is flashed across valve 341 into distillation column 343. Overhead vapor 344 comprising acetic anhydride, methyl acetate, and methyl iodide is condensed, and a portion 345 of this condensate is combined with catalyst makeup 347 and returned to reactor 305. Another portion 346 of this overhead condensate optionally is withdrawn as a mixed product which can be separated by distillation (not shown) to yield the individual products acetic anhydride and methyl acetate, and methyl iodide which is returned to reactor 305.

Bottoms liquid stream 349 comprising EDDA and acetic acid is further separated in distillation column 351 into acetic acid overhead 353 and crude EDDA bottoms 355 containing EDDA and residual heavier components. The crude EDDA is further purified in distillation column 357 to yield high purity EDDA overhead 27 and heavy residue 359. Optionally, EDDA is pyrolyzed and separated as earlier described and shown in Fig. 1 to yield final vinyl acetate product 35 and acetic acid coproduct 10.

The integrated process described above thus enables the production of desired amounts of acetic acid (HOAc), acetic anhydride ($Ac_2O$), ethylidene diacetate (EDDA), and methyl acetate (MeOAc) from a hydrocarbon feed (preferably natural gas), steam, and oxygen without the need for separate production or import of other intermediate components. The additional product vinyl acetate (VAc) can be produced by pyrolysis of EDDA.

There are three important features of the present invention: 1) the direct coupling of the LP DME and LP OA reactors; 2) the optional integration of these reactors with the POX reactor; and 3) the optional overall process to produce VAc. These features are summarized in turn below.

## (1) Direct Coupling of the LP DME and LP OA Process Operations

The LP OA process requires a feed stream consisting of DME, $H_2$ and CO. The typical operating conditions for the LP DME process result in the partial conversion of synthesis gas to DME. One of the key advantages of the present invention is that the feed requirement of the LP OA process directly matches the typical product stream from the LP DME process. The conventional production of EDDA, for example, would typically include the production of DME from methanol, collection and purification of the DME, and finally addition of DME, $H_2$ and CO to an EDDA reactor. In the present invention, by contrast, the unreacted $H_2$ and CO in the gaseous DME product stream from the LP DME reactor becomes the direct feed to the LP OA reactor to produce EDDA and the other coproducts.

The overall net reaction of the synthesis gas feed components for the production of vinyl acetate (VAc) is

$$10\, CO + 7\, H_2 \rightarrow VAc + 2\, HOAc + 2\, CO_2$$

Although this process uses synthesis gas feed in place of more expensive ethylene, it has the disadvantage that it makes 1.4 pounds (kg) of HOAc for each pound (kg) of VAc produced.

If all of the acetic acid is recycled back to the POX unit and combined with natural gas for synthesis gas generation then the actual overall component balance can be approximated as:

$$5.4\, CH_4 + 6.3\, O_2 \rightarrow VAc + 7.8\, H_2O + 1.4\, CO_2$$

If approximately half of the acetic acid is recycled back to the POX unit, the actual overall component balance can be approximated as:

$$6.3\, CH_4 + 5.9\, O_2 \rightarrow VAc + 7.4\, H_2O + 0.1\, CO_2 + 1.1\, HOAc$$

The above equation demonstrates that the carbon balance is very tight if half of the acetic acid is exported as a coproduct. Exporting acetic acid in larger amounts would require that the POX unit be operated on a more carbon-rich feed or that additional hydrogen-rich components (e.g. $H_2$ or MeOH) be exported as coproducts. In such a case, hydrogen would be removed from LP DME reactor feed 13 of Fig. 1.

There are several distinct advantages resulting from the direct coupling of the LP DME and LP OA processes in the present invention compared with existing state-of-the-art technology. These advantages are:

a) EDDA and selected coproducts can be produced directly from readily available and relatively inexpensive synthesis gas. The competing EDDA technologies produce EDDA from more expensive feedstocks including methyl acetate, methanol and DME.

b) There is no requirement for complete synthesis gas conversion to DME. Separate DME production from synthesis gas by known methods requires additional recycle equipment to accomplish nearly total conversion of the feed synthesis gas to DME.

c) Use of the gaseous LP DME product stream eliminates the additional equipment and utilities required to condense and purify the intermediate DME product.

d) Only minimal purification of the methanol byproduct stream is required prior to recycling this liquid byproduct back to the LP DME reactor where it is converted to additional DME.

There are several possible variations to the process within the scope of the present invention. These variations impart flexibility and utility to the process, and can be used for various applications depending on the desired mix of products and available feedstocks. Several of these variations are described in the alternate embodiments described below:

a) The LP DME process is very flexible with respect to byproduct methanol production vs. methanol recycle to the DME reactor. Although the process as described above recycles and consumes all of the byproduct methanol, the process could alternatively produce an exportable methanol product stream or consume excess imported methanol with the feed gas for DME production. This flexibility allows the same installed process equipment to take advantage of the variable market price for methanol. Methanol could be exported during the periods of higher methanol prices and imported during periods of lower methanol prices.

b) The EDDA and acetic acid products are recovered from the LP OA reactor, and other intermediate products are recycled to the reactor along with some of the acetic acid. Alternatively, these other intermediate products can be recovered separately as final products, including methyl acetate, DME, and acetic anhydride. Methyl acetate is a particularly favorable coproduct due to its relatively high concentration in the reactor effluent. This potentially wide product slate from the process is a key advantage of the present invention.

c) As illustrated by the Examples below, the LP OA reactions accommodate the presence of $CO_2$, and the EDDA reaction also partially catalyzes the reverse water gas shift reaction between $CO_2$ and $H_2$ in the feed gas. This reaction ultimately consumes a fraction of the DME, $H_2$, CO and $CO_2$ in the feed gas to produce additional acetic acid. An optimized version of this process may include an additional processing step between the LP DME and the LP OA reactors to reduce the concentration of $CO_2$ in the LP OA reactor feed gas. The added cost and complexity of this additional equipment could be partially offset by higher yields in the LP OA process. Including this step to modify the LP OA feed gas composition would not substantially change the nature of the LP DME and LP OA reactor integration.

(2) Coupling EDDA Production with Synthesis Gas Generation

POX reactors for synthesis gas generation have the flexibility to operate on a wide variety of hydrocarbon feeds and often require the use of a diluent stream (typically steam or $CO_2$) to moderate the reaction combustion temperature. If $CO_2$ is used as the moderant, it can partially react with the available $H_2$ to produce additional CO through the reverse water-gas-shift reaction and thus increase overall carbon utilization.

These characteristics provide a unique opportunity to recycle the coproduct and waste streams from the EDDA production step and convert them into valuable feed streams. Several key advantages which result from the integration of the EDDA synthesis and the POX synthesis gas reactor include:

a) The excess coproduct acetic acid can be used in combination with natural gas as the feed to the POX reactor. This eliminates the need to export excessive quantities of acetic acid and provides a method to maintain the carbon balance of the integrated process since acetic acid is more carbon-rich than natural gas. This integration provides the means to operate the facility in an EDDA-only production mode.

b) The use of excess $CO_2$ vs. steam to moderate POX reactor temperature eliminates the need to import steam in the POX reactor.

c) The use of excess $CO_2$ vs. steam to moderate POX reactor temperature also provides a better carbon utilization. Without this integration, the LP OA reactor offgas would be a waste stream that would be incinerated and vented to the atmosphere. These $CO_2$ molecules are instead converted to synthesis gas which reduces the natural gas feed requirement. The recycled $CO_2$ forms water and CO via the reverse water gas shift reaction in the POX reactor and as a result the waste streams from this process are largely water. This is a clear advantage over emitting a $CO_2$-rich vent stream, since $CO_2$ is considered a greenhouse gas.

d) Recycling unreacted synthesis gas from the LP OA reactor eliminates the need to achieve high synthesis gas conversions per pass. Since the unconverted synthesis gas is largely returned to the process, it is possible to operate the LP OA reactor with an excess of synthesis gas which improves the conversion kinetics of the more valuable DME feed. These improved kinetics ultimately result in a smaller reactor size.

The process as described above maintains an overall carbon balance by recycling nearly all of the acetic acid coproduced in the LP OA reactor. Two other optional methods to maintain or improve the overall carbon balance are to utilize a more carbon-rich POX feed in place of natural gas, and to remove excess hydrogen from LP DME feed [stream 13, Fig. 1]. This hydrogen could be a high value coproduct and would enable the production and export of more acetic acid. This modification to the process would add flexibility and would not substantially change the nature of the present invention.

(3) Overall Integrated Vinyl Acetate Process Scheme

Most of the VAc currently produced is manufactured either by the acetic acid/ethylene route or by the , pyrolysis of EDDA. VAc produced by the acetic acid/ethylene route requires expensive feedstocks while VAc produced by EDDA pyrolysis yields an equimolar amount of acetic acid. The process of the present invention avoids both of these disadvantages. VAc is produced from inexpensive natural gas (or other inexpensive hydrocarbon feedstocks) and the process has the ability to recycle all of the coproduced acetic acid. By having the ability to operate with varying levels of acetic acid recycle, the process operator can vary the amount of acetic acid export to meet market demand.

In addition to converting excess acetic acid into additional synthesis gas, the POX reactor has the capability to convert most of the other small waste and byproduct streams in the overall integrated process to additional synthesis gas feed. This characteristic has both environmental and economic advantages. As a result, the largest waste stream besides coproduced water is the gaseous purge from LP OA offgas which is required to prevent excessive buildup of inert argon and nitrogen gases in the synthesis gas from the POX reactor.

The following examples are presented to further illustrate the present invention.

EXAMPLE 1

A 300 cm$^3$ Hastelloy C autoclave was equipped with a dip tube for loading DME from a preweighed cylinder, a thermocouple, cooling coils, a belt driven magnetic stirrer, and an inlet for gases. The autoclave was protected from overpressure by a rupture disk and a relief valve. All inlet lines, valves and other surfaces being exposed to methyl iodide were made of either Hastelloy C or Inconel. The working volume of the autoclave was approximately 283 cm$^3$.

The following general procedure was used to load, pressurize, run, and unload the autoclave. The autoclave was cooled for 30 minutes by filling with dry ice, the dry ice was removed, and the autoclave was charged with acetic acid, lithium iodide, methyl iodide, lithium acetate, the Group VIII metal component rhodium chloride, and other components given in Tables 1, 3 and 5. The autoclave was sealed, pressurized with nitrogen to test for leaks, vented, pressurized with a premixed synthesis gas containing 70 vol% CO/30% vol% H$_2$ at least thrice, and vented to approximately 20 psig (140 kPa). DME was transferred to the autoclave from a preweighed cylinder. While stirring, the synthesis gas pressure was increased to 300-400 psig (2-2.75 MPa) from a ballast. The ballast pressure was recorded and the reactor was heated to operating temperature. At operating temperature, reactor pressure was increased to operating pressure from the ballast. The reactions were carried out for the desired length of time while the autoclave was maintained at constant pressure. Following completion of the reaction, the autoclave was cooled to room temperature and a valve leading to a flash pot capture cylinder (500 cm$^3$) was opened. The autoclave contents were flashed into the capture cylinder and the resulting pressure was recorded. The flash liquid and vapor in the capture cylinder were analyzed by gas chromatography using a DB-1701 FSOT capillary column interfaced to a flame ionization detector. Quantitation was obtained using an internal standard technique, and the lower detection limit for the compounds of interest was approximately 0.002 wt.%. All organic compound structures were verified by gas chromatography/mass spectrometry (GC/MS).

The operating conditions, feed component weights, and flash liquid component weights are summarized in Table 1.

Table 1

| Results of Autoclave Run No. 1 | | |
|---|---|---|
| Reaction Conditions: | Temperature | 374°F (190°C) |
| | Pressure | 1443 psig (10.05 MPa) |
| | Reaction time | 90 min |
| | Weight, grams | |
| | Initial Reactor Charge | Flash Pot Liquid |
| Component | | |
| Carbon dioxide | 8.00 | --- |
| Dimethyl ether | 19.00 | 0.31 |
| Methanol | 2.11 | --- |
| Water | 0.33 | --- |
| Acetic acid | 129.44 | 147.90 |
| Ethylidene diacetate | --- | 10.38 |
| Acetaldehyde | --- | 0.08 |
| Methyl acetate | --- | 18.62 |
| Acetic anhydride | 13.91 | 4.10 |
| Methyl iodide | 8.48 | 5.42 |
| Lithium iodide | 1.50 | --- |
| Rhodium chloride | 0.30 | --- |
| Lithium acetate | 0.99 | --- |
| Total weight, grams | 184.06 | 194.80 |

These experimental data were used with predicted phase equilibria and material balances to calculate the vapor and liquid compositions for the charged and heated reactor at initial reaction conditions, the reactor at final reaction conditions, and the flash pot at ambient temperature. The results of the calculations are summarized in Table 2 and indicate the relative distribution of components between the vapor and liquid phases. DME conversions to the various coproduct components are also given in Table 2.

TABLE 2

| Calculated Liquid and Vapor Compositions for Run No. 1 | | | | | | |
|---|---|---|---|---|---|---|
| | Initial Reaction Conditions | | Final Reaction Conditions | | Flash Pot Split | |
| | Liquid | Vapor | Liquid | Vapor | Liquid | Vapor |
| Temperature (°F) | 374 | 374 | 374 | 374 | 77 | 77 |
| (°C) | 190 | 190 | 190 | 190 | 25 | 25 |
| Pressure (psia) | 1458 | 1458 | 1458 | 1458 | 263 | 263 |
| (MPa) | 10.05 | 10.05 | 10.05 | 10.05 | 1.81 | 1.81 |
| Volume ($cm^3$) | 236.1 | 46.9 | 244.6 | 38.4 | | |
| Total Charge (grams) | 181.2 | 4.1 | 197.1 | 1.8 | 194.4 | 8.5 |
| (mgmoles) | 3098 | 129 | 3124 | 96 | 2985 | 434 |
| | | | | | | |
| Composition | (wt%) | (mol%) | (wt%) | (mol%) | (wt%) | (mol%) |
| Hydrogen | 0.03 | 21.75 | 0.07 | 57.46 | 0.002 | 41.32 |
| Carbon Monoxide | 1.46 | 33.52 | 0.68 | 19.35 | 0.130 | 45.27 |
| Carbon Dioxide | 3.87 | 17.23 | 2.31 | 13.18 | 1.37 | 12.87 |
| Dimethyl Ether | 9.92 | 17.02 | 0.17 | 0.28 | 0.16 | 0.14 |
| Methanol | 1.14 | 0.94 | 0 | 0 | 0 | 0 |
| Water | 0.18 | 0.18 | 0 | 0 | 0 | 0 |
| Acetic Acid | 71.15 | 6.84 | 74.91 | 5.29 | 76.08 | 0.0213 |
| Ethylidene diacetate | | | 5.34 | 0.11 | 5.42 | 0.0007 |
| Acetaldehyde | | | 0.05 | 0.04 | 0.05 | 0.0064 |
| Methyl Acetate | | | 9.95 | 2.62 | 10.15 | 0.1962 |
| Acetic Anhydride | 7.64 | 0.55 | 2.26 | 0.12 | 2.30 | 0.0005 |
| Methyl Iodide | 4.60 | 0.80 | 4.26 | 0.64 | 4.34 | 0.07 |
| Total Collected Weight of Liq+Vap+Cat (grams) | 188.0 | | 201.7 | | 200.9 | |
| | | | | | | |
| DME Conversions, %: | | | | | | |
| Ethylidene diacetate | 35.00 | | | | | |
| Methyl Acetate | 64.79 | | | | | |
| Acetic Anhydride | -22.41 | | | | | |
| Acetaldehyde | 0.53 | | | | | |
| From $CO_2$ Shift | 15.87 | | | | | |
| From Water | 4.44 | | | | | |
| Total Percent Conversion | 98.22 | | | | | |
| | | | | | | |
| Average Productivities: | 4.52 mol DME/(mol $CH_3I\cdot h$) | | | | | |
| | 0.81 mol EDDA/(mol $CH_3I\cdot h$) | | | | | |

The calculated dimethyl ether conversion was 4.52 mol DME per mole methyl iodide per hour, and the EDDA reactor productivitywas 0.81 mole EDDA per mole methyl iodide catalyst per hour. The addition of carbon dioxide, methanol, and water to the reactor feed simulates the direct flow of LP DME reactor effluent into the LP EDDA reactor without intermediate methanol or water removal, and confirms that EDDA synthesis can be carried out successfully under these conditions. Acetic anhydride, an intermediate product of EDDA synthesis, can be recycled to the reactor where it becomes an intermediate reactant for additional EDDA synthesis. Acetic acid, which is added to the reactor as the main component of the liquid phase, is both a reactant and a net product in the overall reaction mechanism.

EXAMPLE 2

The procedure of Example 1 was utilized to test the reaction characteristics at a lower pressure (1007 psig; 7.047 MPa) and a shorter reaction time (45 min) using different amounts of catalyst charge. The results are summarized in Table 3.

Table 3

| Results of Autoclave Run No. 2 | | |
| --- | --- | --- |
| Reaction Conditions: | Temperature<br>Pressure<br>Reaction time | 374°F (190°C)<br>1007 psig (7.047 MPa)<br>45 min |
| | Weight, grams | |
| | Initial Reactor Charge | Flash Liquid |
| Component | | |
| Carbon dioxide | 4.50 | --- |
| Dimethyl ether | 20.96 | 3.36 |
| Methanol | 0.74 | --- |
| Water | 0.07 | --- |
| Acetic acid | 128.90 | 147.34 |
| Ethylidene diacetate | --- | 4.95 |
| Acetaldehyde | --- | 0.04 |
| Methyl acetate | --- | 22.06 |
| Acetic anhydride | 17.51 | 8.38 |
| Methyl iodide | 12.97 | 7.50 |
| Lithium iodide | 1.50 | --- |
| Rhodium chloride | 0.40 | --- |
| Lithium acetate | 1.99 | --- |
| Total weight, grams | 189.54 | 196.30 |

These experimental data were used with predicted phase equilibria and material balances to calculate the vapor and liquid compositions for the charged and heated reactor at initial reaction conditions, the reactor at final reaction conditions, and the flash pot at ambient temperature. The results of the calculations are summarized in Table 4 and indicate the relative distribution of components between the vapor and liquid phases. DME conversions to the various coproduct components are also given in Table 4.

TABLE 4

| | Calculated Liquid and Vapor Compositions for Run No. 2 | | | | | |
| --- | --- | --- | --- | --- | --- | --- |
| | Initial Reaction Conditions | | Final Reaction Conditions | | Flash Pot Split | |
| | Liquid | Vapor | Liquid | Vapor | Liquid | Vapor |
| Temperature (°F) | 374 | 374 | 374 | 374 | 77 | 77 |
| (°C) | 190 | 190 | 190 | 190 | 25 | 25 |
| Pressure (psia) | 1022 | 1022 | 1022 | 1022 | 177 | 177 |
| (MPa) | 7.047 | 7.047 | 7.047 | 7.047 | 1.220 | 1.220 |
| Volume (cm$^3$) | 242.9 | 40.1 | 248.8 | 34.3 | | |
| Total Charge (grams) | 185.4 | 2.7 | 197.4 | 1.3 | 196.1 | 5.5 |
| (mgmoles) | 3037 | 78 | 3077 | 61 | 2988 | 289 |
| Composition | (wt%) | (mol%) | (wt%) | (mol%) | (wt%) | (mol%) |
| Hydrogen | 0.02 | 20.09 | 0.04 | 50.88 | 0.001 | 39.60 |
| Carbon Monoxide | 0.93 | 29.56 | 0.74 | 28.23 | 0.111 | 54.73 |
| Carbon Dioxide | 2.18 | 13.06 | 0.36 | 2.80 | 0.21 | 2.92 |

TABLE 4   (continued)

| Calculated Liquid and Vapor Compositions for Run No. 2 | | | | | | |
|---|---|---|---|---|---|---|
| | Initial Reaction Conditions | | Final Reaction Conditions | | Flash Pot Split | |
| | Liquid | Vapor | Liquid | Vapor | Liquid | Vapor |
| Dimethyl Ether | 10.82 | 24.17 | 1.78 | 4.02 | 1.71 | 2.10 |
| Methanol | 0.39 | 0.41 | 0 | 0 | 0 | 0 |
| Water | 0.04 | 0.05 | 0 | 0 | 0 | 0 |
| Acetic Acid | 69.30 | 8.17 | 72.32 | 6.73 | 72.94 | 0.0277 |
| Ethylidene diacetate | | | 2.51 | 0.06 | 2.53 | 0.0004 |
| Acetaldehyde | | | 0.02 | 0.02 | 0.02 | 0.0041 |
| Methyl Acetate | | | 11.47 | 3.81 | 11.61 | 0.2824 |
| Acetic Anhydride | 9.41 | 0.82 | 4.25 | 0.30 | 4.29 | 0.0013 |
| Methyl Iodide | 6.90 | 1.50 | 6.51 | 1.28 | 6.59 | 0.14 |
| Total Collected Weight of Liq+Vap+Cat (grams) | 192.0 | | 202.6 | | 202.3 | |
| | | | | | | |
| DME Conversions, %: | | | | | | |
| Ethylidene diacetate | 14.92 | | | | | |
| Methyl Acetate | 67.70 | | | | | |
| Acetic Anhydride | -19.60 | | | | | |
| Acetaldehyde | 0.23 | | | | | |
| From $CO_2$ Shift | 18.57 | | | | | |
| From Water | 0.85 | | | | | |
| Total Percent Conversion | 82.67 | | | | | |
| | | | | | | |
| Average Productivities: | 5.49 mol DME/(mol $CH_3I \cdot h$) | | | | | |
| | 0.50 mol EDDA/(mol $CH_3I \cdot h$) | | | | | |

The calculated dimethyl ether conversion was 5.49 mol DME per mole methyl iodide catalyst per hour and the EDDA reactor productivity was 0.50 mole EDDA per mole methyl iodide per hour. The addition of carbon dioxide, methanol, and water to the reactor feed again confirmed that EDDA synthesis can be carried out successfully using LP DME reactor effluent as direct feed to the EDDA reactor.

EXAMPLE 3

The procedure of Example 2 was utilized to test the reaction characteristics when the intermediate product methyl acetate was added to the feed to simulate recycle of this component to the reactor. The results are summarized in Table 5 below.

Table 5

| Results of Autoclave Run No. 3 | | |
|---|---|---|
| | | |
| Reaction Conditions: | Temperature | 374°F (190°C) |
| | Pressure | 989 psig (6.923 MPa) |
| | Reaction time | 45 min |
| | Weight, grams | |
| | Initial Reactor Charge | Flash Liquid |
| Component | | |
| Carbon dioxide | 5.47 | ??? |

Table 5   (continued)

| Results of Autoclave Run No. 3 | | |
| --- | --- | --- |
| | Weight, grams | |
| | Initial Reactor Charge | Flash Liquid |
| Component | | |
| Dimethyl ether | 25.40 | 11.29 |
| Methanol | 0.70 | --- |
| Water | 0.05 | --- |
| Acetic acid | 115.00 | 135.04 |
| Ethylidene diacetate | --- | 3.56 |
| Acetaldehyde | --- | 0.57 |
| Methyl acetate | 14.96 | 31.58 |
| Acetic anhydride | 14.10 | 7.98 |
| Methyl iodide | 13.00 | 9.27 |
| Lithium iodide | 1.50 | --- |
| Rhodium chloride | 0.40 | --- |
| Lithium acetate | 2.06 | --- |
| | | |
| Total weight, grams | 192.64 | 198.00 |

These experimental data were used with predicted phase equilibria and material balances to calculate the vapor and liquid compositions for the charged and heated reactor at initial reaction conditions, the reactor at final reaction conditions, and the flash pot at ambient temperature. The results of the calculations are summarized in Table 4 and indicate the relative distribution of components between the vapor and liquid phases. DME conversions to the various coproduct components are also given in Table 6.

TABLE 6

| Calculated Liquid and Vapor Compositions for Run No. 3 | | | | | | |
| --- | --- | --- | --- | --- | --- | --- |
| | Initial Reaction Conditions | | Final Reaction Conditions | | Flash Pot Split | |
| | Liquid | Vapor | Liquid | Vapor | Liquid | Vapor |
| Temperature (°F) | 374 | 374 | 374 | 374 | 77 | 77 |
| (°C) | 190 | 190 | 190 | 190 | 25 | 25 |
| Pressure (psia) | 1004 | 1004 | 1004 | 1004 | 199 | 199 |
| (MPa) | 6.923 | 6.923 | 6.923 | 6.923 | 1.372 | 1.372 |
| Volume (cm$^3$) | 236.9 | 46.1 | 242.5 | 40.5 | | |
| Total Charge (grams) | 187.0 | 3.7 | 197.3 | 2.3 | 195.2 | 7.6 |
| (mgmoles) | 3053 | 95 | 3111 | 76 | 3015 | 333 |
| | | | | | | |
| Composition | (wt%) | (mol%) | (wt%) | (mol%) | (wt%) | (mol%) |
| Hydrogen | 0.02 | 14.91 | 0.03 | 35.77 | 0.001 | 32.24 |
| Carbon Monoxide | 0.74 | 22.76 | 0.66 | 24.14 | 0.120 | 50.96 |
| Carbon Dioxide | 2.54 | 17.21 | 1.20 | 10.02 | 0.66 | 9.73 |
| Dimethyl Ether | 12.87 | 30.43 | 5.90 | 14.27 | 5.71 | 6.44 |
| Methanol | 0.37 | 0.44 | 0 | 0 | 0 | 0 |
| Water | 0.03 | 0.04 | 0 | 0 | 0 | 0 |
| Acetic Acid | 61.24 | 8.35 | 63.96 | 7.30 | 64.79 | 0.0243 |
| Ethylidene diacetate | | | 1.73 | 0.06 | 1.75 | 0.0003 |
| Acetaldehyde | | | 0.33 | 0.37 | 0.33 | 0.0568 |
| Methyl Acetate | 7.87 | 3.45 | 15.80 | 6.25 | 16.09 | 0.3961 |
| Acetic Anhydride | 7.50 | 0.76 | 3.87 | 0.34 | 3.92 | 0.0012 |

TABLE 6   (continued)

| Calculated Liquid and Vapor Compositions for Run No. 3 | | | | | | |
|---|---|---|---|---|---|---|
| | Initial Reaction Conditions | | Final Reaction Conditions | | Flash Pot Split | |
| | Liquid | Vapor | Liquid | Vapor | Liquid | Vapor |
| Methyl Iodide | 6.83 | 1.64 | 6.51 | 1.48 | 6.62 | 0.15 |
| Total Collected Weight of Liq+Vap+Cat (grams) | 194.7 | | 203.5 | | 202.5 | |
| DME Conversions, %: | | | | | | |
| Ethylidene diacetate | 8.49 | | | | | |
| Methyl Acetate | 40.55 | | | | | |
| Acetic Anhydride | -11.44 | | | | | |
| Acetaldehyde | 2.71 | | | | | |
| From $CO_2$ Shift | 11.40 | | | | | |
| From Water | 0.50 | | | | | |
| Total Percent Conversion | $\overline{52.22}$ | | | | | |
| Average Productivities: | 4.19 mol DME/(mol $CH_3I·h$) | | | | | |
| | 0.34 mol EDDA/(mol $CH_3I·h$) | | | | | |

The calculated dimethyl ether conversion was 4.19 mole DME per mole methyl iodide catalyst per hour and the EDDA reactor productivity was 0.34 mole EDDA per mole methyl iodide per hour. The addition of carbon dioxide, methanol, and water to the reactor feed again confirmed that EDDA synthesis can be carried out successfully using LP DME reactor effluent as direct feed to the EDDA reactor without the need for intermediate separation steps. The addition of methyl acetate to the feed to simulate recycle of this intermediate compound may have been a factor in the reduced EDDA reactor productivity compared with Example 2, but indicates that recycle of methyl acetate is feasible.

EXAMPLE 4

Material balances were prepared for the production of vinyl acetate (VAc) at a rate of 755 lb moles/hour (342 kg moles/h) according to the embodiment of Fig. 1 using natural gas as feed to the partial oxidation (POX) synthesis gas reactor 101. In a first material balance, 50% of the total acetic acid stream 43 was taken as product 10 and the remainder 9 was sent to POX reactor 101 to generate additional synthesis gas. Table 7 summarizes the stream conditions and properties for the 50% recycle case, and shows that a natural gas feed rate (as methane) of 4790 lb moles/hour (2175 kg moles/h) is required to produce 755 lb moles/h (342 kg moles/h) of vinyl acetate with a coproduct acetic acid rate of 804 lb moles/h (365 kg moles/h). A second material balance was prepared for the recycle of 100% of the acetic acid to the POX reaction system for the same vinyl acetate production rate of 755 lb moles/hour as summarized in Table 8.

TABLE 7

| Material Balance for 50% Acetic Acid Recycle | | | | | | | | | |
|---|---|---|---|---|---|---|---|---|---|
| Material Balance Point | 1 | 5 | 7 | 9 | 10 | 11 | 13 | 19 | 21 |
| Temperature (°F) | 80 | 80 | 100 | 100 | 100 | 100 | 100 | 280 | 170 |
| (°C) | 27 | 27 | 38 | 38 | 38 | 38 | 38 | 138 | 77 |
| Pressure (psia) | 1250 | 1250 | 1050 | 1250 | 20 | 1200 | 1200 | 1150 | 20 |
| (MPa) | 8.60 | 8.60 | 7.25 | 8.60 | 0.14 | 8.25 | 8.25 | 7.95 | 0.14 |
| Average Molecular Wt. | 16.0 | 32.0 | 36.1 | 60.1 | 60.1 | 18.0 | 22.0 | 33.0 | 32.0 |
| **Component (mol/h)** | | | | | | | | | |
| Hydrogen | | | 848 | | | | 6557 | 1629 | |
| Carbon Monoxide | | | 905 | | | | 8663 | 4050 | |
| Carbon Dioxide | | | 4244 | | | | 2942 | 4375 | |
| Argon | | 23 | 737 | | | | 760 | 760 | |

TABLE 7   (continued)

| Material Balance for 50% Acetic Acid Recycle | | | | | | | | | |
|---|---|---|---|---|---|---|---|---|---|
| Material Balance Point | 1 | 5 | 7 | 9 | 10 | 11 | 13 | 19 | 21 |
| Oxygen | | 4543 | | | | | | | |
| Methanol | | | | | | | | 462 | 385 |
| Water | | | | | | 5567 | | 118 | |
| Methane | 4790 | | 27 | | | | 28 | 28 | |
| Dimethyl Ether | | | 30 | | | | | 1551 | |
| Acetic Acid | | | | 804 | 804 | | | | |
| EDDA | | | | | | | | | |
| VAc | | | | | | | | | |
| Total lbmol/h | 4790 | 4565 | 6791 | 804 | 804 | 5567 | 18950 | 12974 | 385 |
| kgmol/h | 2173 | 2071 | 3080 | 365 | 365 | 2525 | 8596 | 5885 | 175 |
| Material Balance Point | 22 | 23 | 25 | 27 | 29 | 31 | 33 | 39 | 41 |
| Temperature (°F) | 170 | 100 | 100 | 100 | 100 | 100 | 100 | 100 | 100 |
| (°C) | 77 | 38 | 38 | 38 | 38 | 38 | 38 | 38 | 38 |
| Pressure (psia) | 20 | 20 | 1150 | 20 | 20 | 1050 | 1050 | 15 | 15 |
| (MPa) | 0.14 | 0.14 | 7.95 | 0.14 | 0.14 | 7.25 | 7.25 | 0.10 | 0.10 |
| Average Molecular Wt. | 32.0 | 18.0 | 33.2 | 146.1 | 60.1 | 36.1 | 36.1 | 60.1 | 86.1 |
| **Component (mol/h)** | | | | | | | | | |
| Hydrogen | | | 1629 | | | 874 | 26 | | |
| Carbon Monoxide | | | 4050 | | | 933 | 28 | | |
| Carbon Dioxide | | | 4375 | | | 4375 | 131 | | |
| Argon | | | 760 | | | 760 | 23 | | |
| Oxygen | | | | | | | | | |
| Methanol | 384 | | 77 | | | | | | |
| Water | | 108 | 10 | | | | | | |
| Methane | | | 28 | | | 28 | 0.8 | | |
| Dimethyl Ether | | | 1551 | | | 31 | 0.9 | | |
| Acetic Acid | | | | | 852 | | | 755 | |
| EDDA | | | | 755 | | | | | |
| VAc | | | | | | | | | 755 |
| Total lbmol/h | 384 | 108 | 12481 | 755 | 852 | 7001 | 210 | 755 | 755 |
| kgmol/h | 174 | 49 | 5661 | 342 | 386 | 3176 | 95 | 342 | 342 |

TABLE 8

| Material Balance for 100% Acetic Acid Recycle | | | | | | | | | |
|---|---|---|---|---|---|---|---|---|---|
| Material Balance Point | 1 | 5 | 7 | 9 | 10 | 11 | 13 | 19 | 21 |
| Temperature (°F) | 80 | 80 | 100 | 100 | 100 | 100 | 100 | 280 | 170 |
| (°C) | 27 | 27 | 38 | 38 | 38 | 38 | 38 | 138 | 77 |
| Pressure (psia) | 1250 | 1250 | 1050 | 1250 | 20 | 1200 | 1200 | 1150 | 20 |
| (MPa) | 8.60 | 8.60 | 7.25 | 8.60 | 0.14 | 8.25 | 8.25 | 7.95 | 0.14 |
| Average Molecular Wt. | 16.0 | 32.0 | 34.9 | 60.1 | 60.1 | 18.0 | 21.1 | 32.3 | 32.0 |
| **Component (mol/h)** | | | | | | | | | |
| Hydrogen | | | 813 | | | | 6697 | 1769 | |
| Carbon Monoxide | | | 748 | | | | 8663 | 4050 | |
| Carbon Dioxide | | | 3450 | | | | 2870 | 4303 | |
| Argon | | 23 | 92 | | | | 115 | 115 | |

TABLE 8   (continued)

| Material Balance for 100% Acetic Acid Recycle | | | | | | | | | |
|---|---|---|---|---|---|---|---|---|---|
| Material Balance Point | 1 | 5 | 7 | 9 | 10 | 11 | 13 | 19 | 21 |
| Oxygen | | 4532 | | | | | | | |
| Methanol | | | | | | | | 462 | 385 |
| Water | | | | | | 5548 | | 118 | |
| Methane | 4077 | | 22 | | | | 28 | 28 | |
| Dimethyl Ether | | | 25 | | | | | 1551 | |
| Acetic Acid | | | | 1607 | 0 | | | | |
| EDDA | | | | | | | | | |
| VAc | | | | | | | | | |
| Total lbmol/h | 4077 | 4555 | 5151 | 1607 | 0 | 5548 | 18373 | 12396 | 385 |
| kgmol/h | 1849 | 2066 | 2336 | 729 | 0 | 2517 | 8334 | 5623 | 175 |

| Material Balance Point | 22 | 23 | 25 | 27 | 29 | 31 | 33 | 39 | 41 |
|---|---|---|---|---|---|---|---|---|---|
| Temperature (°F) | 170 | 100 | 100 | 100 | 100 | 100 | 100 | 100 | 100 |
| (°C) | 77 | 38 | 38 | 38 | 38 | 38 | 38 | 38 | 38 |
| Pressure (psia) | 20 | 20 | 1150 | 20 | 20 | 1050 | 1050 | 15 | 15 |
| (MPa) | 0.14 | 0.14 | 7.95 | 0.14 | 0.14 | 7.25 | 7.25 | 0.10 | 0.10 |
| Average Molecular Wt. | 32.0 | 18.0 | 32.4 | 146.1 | 60.1 | 34.9 | 34.9 | 60.1 | 86.1 |
| **Component (mol/h)** | | | | | | | | | |
| Hydrogen | | | 1769 | | | 1014 | 201 | | |
| Carbon Monoxide | | | 4050 | | | 933 | 185 | | |
| Carbon Dioxide | | | 4303 | | | 4303 | 853 | | |
| Argon | | | 115 | | | 115 | 23 | | |
| Oxygen | | | | | | | | | |
| Methanol | 384 | | 77 | | | | | | |
| Water | | 108 | 10 | | | | | | |
| Methane | | | 28 | | | 28 | 5.5 | | |
| Dimethyl Ether | | | 1551 | | | 31 | 6.1 | | |
| Acetic Acid | | | | | 852 | | | 755 | |
| EDDA | | | | 755 | | | | | |
| VAc | | | | | | | | | 755 |
| Total lbmol/h | 384 | 108 | 11903 | 755 | 852 | 6424 | 1273 | 755 | 755 |
| kgmol/h | 174 | 49 | 5399 | 342 | 386 | 2914 | 577 | 342 | 342 |

It is seen from Tables 7 and 8 that the required natural gas feed rate is reduced by 713 lb moles/hour (323 kgmole/h) or 15% by recycling the additional acetic acid coproduct to the POX reactor. Thus the recycle of acetic acid to the POX reactor for conversion into additional synthesis gas is a useful alternative when there is no market for the coproduct acetic acid.

The present invention thus allows the production of oxygenated acetyl compounds directly from synthesis gas via the liquid phase dimethyl ether process. Several variations to the disclosed process are possible within the scope of the present invention. These variations impart flexibility and utility to the process, and can be used in alternative applications depending on the desired mix of products and available feedstocks. The product slate can include one or more of the oxygenated acetyl compounds vinyl acetate, ethylidene diacetate, acetic acid, methyl acetate, and acetic anhydride. Unwanted coproducts are conveniently recycled to the POX reactor which reduces the POX feed requirement.

**Claims**

1.   A process for the synthesis of oxygenated acetyl compounds from synthesis gas comprising hydrogen and carbon monoxide, wherein said process comprises:

16

(a) reacting said synthesis gas in a first liquid phase reactor in the presence of a methanol synthesis catalyst and a methanol dehydration catalyst suspended in an inert liquid to produce dimethyl ether and methanol;

(b) withdrawing from said first reactor an intermediate product stream comprising dimethyl ether, methanol, water, and unreacted synthesis gas;

(c) passing said intermediate stream into a second liquid phase reactor and reacting said dimethyl ether, methanol, and unreacted synthesis gas with acetic acid in the presence of a catalyst system consisting essentially of a Group VIII metal, methyl iodide, lithium iodide and, optionally, lithium acetate to produce one or more oxygenated acetyl compounds selected from ethylidene diacetate, acetic acid, acetic anhydride, and methyl acetate; and

(d) withdrawing from said second liquid phase reactor a liquid product mixture comprising said one or more oxygenated acetyl compounds and a vapor stream comprising unreacted synthesis gas.

2. A process as claimed in Claim 1, wherein said synthesis gas is produced by the partial oxidation of a carbonaceous feedstock in a partial oxidation reactor system.

3. A process as claimed in Claim 2, wherein said carbonaceous feedstock is natural gas.

4. A process as claimed in any one of the preceding claims, wherein said methanol synthesis catalyst is a Cu/ZnO/Al$_2$O$_3$ catalyst.

5. A process as claimed in any one of the preceding claims, wherein said methanol dehydration catalyst is alumina obtained by heat treating boehmite.

6. A process as claimed in any one of the preceding claims, wherein said methanol synthesis catalyst comprises copper and said methanol dehydration catalyst comprises alumina, wherein the methanol synthesis catalyst is between 75 and 90% of the methanol synthesis catalyst plus methanol dehydration catalyst on a weight basis.

7. A process as claimed in any one of the preceding claims, wherein said first liquid phase reactor is operated at a temperature between 225 and 270°C (440 and 520°F), a pressure between 5 and 14 MPa (750 and 2,000 psig), and a gas hourly space velocity of between 3,000 and 15,000 standard liters/(kg catalyst-h).

8. A process as claimed in any one of the preceding claims, which further comprises separating methanol and water from said intermediate product stream by distillation, and recycling at least a portion of the separated methanol to said first liquid phase reactor.

9. A process as claimed in any one of the preceding claims, wherein said Group VIII metal is rhodium (III) chloride trihydrate.

10. A process as claimed in any one of the preceding claims, wherein said catalyst system further comprises lithium acetate.

11. A process as claimed in any one of the preceding claims, wherein the liquid in said second liquid phase reactor comprises one or more of said oxygenated acetyl compounds.

12. A process as claimed in any one of the preceding claims, wherein said second liquid phase reactor is operated at a temperature between 120° and 195°C and a pressure between 3 to 15 MPa (400 and 2100 psig).

13. A process as claimed in any one of the preceding claims, wherein ethylidene diacetate and acetic acid are separated by distillation of said liquid product mixture.

14. A process as claimed in Claim 13 which further comprises recycling a first portion of said acetic acid to said second liquid phase reactor.

15. A process as claimed in Claim 14, wherein said synthesis gas is produced by the partial oxidation of a carbonaceous feedstock in a partial oxidation reactor system, and wherein a second portion of said acetic acid is recycled to said partial oxidation reactor system.

16. A process as claimed in Claim 15 which further comprises recycling at least a portion of unreacted from said second liquid phase reactor synthesis gas to said partial oxidation reactor system.

17. A process as claimed in any one of Claims 13 to 16 which further comprises passing said ethylidene diacetate into a pyrolysis reactor system wherein said ethylidene diacetate is converted into an intermediate product comprising vinyl acetate and acetic acid, and separating said intermediate product by distillation into vinyl acetate and acetic acid.

18. A process as claimed in Claim 17, wherein said synthesis gas is produced by the partial oxidation of a carbonaceous feedstock in a partial oxidation reactor system, and at least a portion of said acetic acid separated from said intermediate product is recycled to said partial oxidation reactor system.

19. A process as claimed in any one of the preceding claims, which further comprises separating a mixture comprising acetic anhydride and methyl acetate from the liquid product mixture from said second liquid phase reactor, and recycling at least a portion of said mixture to said second liquid phase reactor.

20. A process as claimed in any one of the preceding claims, wherein said unreacted synthesis gas from said first liquid phase reactor further comprises carbon dioxide, and further wherein at least a portion of the carbon dioxide in said unreacted synthesis gas is removed prior to said second liquid phase reactor.

21. A process as claimed in any one of the preceding claims, wherein at least a portion of the hydrogen is removed from said unreacted synthesis gas from said first liquid phase reactor prior to said second liquid phase reactor, thereby increasing the production of acetic anhydride and methyl acetate and decreasing the production of ethylidene diacetate and acetic acid.

**Patentansprüche**

1. Verfahren zur Herstellung von oxygenierten Acetylverbindungen aus Synthesegas, welches Wasserstoff und Kohlenmonoxid enthält, bei dem:

   (a) das Synthesegas in einem ersten Flüssigphasenreaktor in Anwesenheit eines Methanol-Synthesekatalysators und eines Methanol-Dehydrierungskatalysators, die in einer inerten Flüssigkeit suspendiert sind, umgesetzt wird, um Dimethylether und Methanol herzustellen,
   (b) ein Zwischenproduktstrom, welcher Dimethylether, Methanol, Wasser und nicht umgesetztes Synthesegas enthält, aus dem ersten Reaktor entnommen wird
   (c) der Zwischenproduktstrom in einen zweiten Flüssigphasenreaktor geleitet und der Dimethylether, das Methanol und nicht umgesetztes Synthesegas mit Essigsäure in Anwesenheit eines Katalysatorsystems umgesetzt werden, das im wesentlichen aus einem Metall der Gruppe VIII, Methyliodid, Lithiumiodid und gegebenenfalls Lithiumacetat besteht, um ein oder mehrere oxygenierte Acetylverbindungen, ausgewählt unter Ethylidendiacetat, Essigsäure, Essigsäureanhydrid und Methylacetat herzustellen, und
   (d) aus dem zweiten Flüssigphasenreaktor ein flüssiges Produktgemisch entnommen wird, das ein oder mehrere oxygenierte Acetylverbindungen und einen Gasstrom umfaßt, der nicht umgesetztes Synthesegas enthält.

2. Verfahren nach Anspruch 1, bei dem Synthesegas durch teilweise Oxydation einer kohlenstoffhaltigen Quelle in einem Teiloxidations-Reaktorsystem hergestellt wird.

3. Verfahren nach Anspruch 2, bei dem die kohlenstoffhaltige Quelle Erdgas ist.

4. Verfahren nach einem der vorhergehenden Ansprüche, bei dem der Methanol-Synthesekatalysator ein Cu/ZnO/Al$_2$O$_3$-Katalysator ist.

5. Verfahren nach einem der vorhergehenden Ansprüche, bei dem der Methanol-Dehydrierungskatalysator Aluminiumoxid ist, das durch Wärmebehandlung von Boehmit erhalten wurde.

**6.** Verfahren nach einem der vorhergehenden Ansprüche, bei dem der Methanol-Synthesekatalysator Kupfer enthält und der Methanol-Deydrierungskatalysator Aluminiumoxid enthält, wobei der Methanol-Synthesekatalysator zwischen 75 und 90 % der Summe von Methanol-Synthesekatalysator und Methanol-Dehydrierungskatalysator, bezogen auf das Gewicht, vorhanden ist.

**7.** Verfahren nach einem der vorhergehenden Ansprüche, wobei der erste Flüssigphasenreaktor bei einer Temperatur zwischen 225 und 270°C (440 und 520°F), einem Druck zwischen 5 und 14 MPa (750 und 2.000 Psig) und einer Gasraumgeschwindigkeit pro Stunde von zwischen 3.000 und 15.000 Standardliter/(kg Katalysator Std.) betrieben wird.

**8.** Verfahren nach einem der vorhergehenden Ansprüche, bei dem weiter Methanol und Wasser von dem Zwischenproduktstrom mittels Destillation abgetrennt wird und mindestens ein Teil des abgetrennten Methanols in den ersten Flüssigphasenreaktor zurückgeführt wird.

**9.** Verfahren nach einem der vorhergehenden Ansprüche, bei dem das Metall der Gruppe VIII Rhodium(III)chlorid-Trihydrat ist.

**10.** Verfahren nach einem der vorhergehenden Ansprüche, bei dem das Katalysatorsystem zusätzlich Lithiumacetat enthält.

**11.** Verfahren nach einem der vorhergehenden Ansprüche, bei dem die Flüssigkeit in dem zweiten Flüssigphasenreaktor ein oder mehrere der oxygenierten Acetylverbindungen enthält.

**12.** Verfahren nach einem der vorhergehenden Ansprüche, bei dem der zweite Flüssigphasenreaktor bei einer Temperatur zwischen 120° und 195°C und einem Druck zwischen 3 bis 15 MPa (400 und 2.100 Psig) betrieben wird.

**13.** Verfahren nach einem der vorhergehenden Ansprüche, bei dem das Ethylidendiacetat und die Essigsäure mittels Destillation von dem flüssigen Produktgemisch abgetrennt werden.

**14.** Verfahren nach Anspruch 13, bei dem weiter ein erster Teil der Essigsäure in den zweiten Flüssigphasenreaktor zurückgeführt wird.

**15.** Verfahren nach Anspruch 14, bei dem das Synthesegas durch teilweise Oxydation einer kohlenstoffhaltigen Quelle in einem Teiloxydations-Reaktorsystem hergestellt wird und wobei ein zweiter Teil der Essigsäure in das Teiloxydations-Reaktorsystem zurückgeführt wird.

**16.** Verfahren nach Anspruch 15, bei dem weiter mindestens ein Teil des nicht umgesetzten Synthesegases aus dem zweiten Flüssigphasenreaktor in das Teiloxydations-Reaktorsystem zurückgeführt wird.

**17.** Verfahren nach einem der Ansprüche 13 bis 16, bei dem weiter das Ethylidendiacetat in ein Pyrolysereaktorsystem geführt wird, in dem das Ethylidendiacetat in ein Zwischenprodukt umgewandelt wird, das Vinylacetat und Essigsäure enthält und das Zwischenprodukt mittels Destillation in Vinylacetat und Essigsäure aufgetrennt wird.

**18.** Verfahren nach Anspruch 17, bei dem das Synthesegas durch teilweise Oxydation einer kohlenstoffhaltigen Quelle in einem Teiloxydations-Reaktorsystem hergestellt wird und mindestens ein Teil der Essigsäure des Zwischenproduktes in das Teiloxydations-Reaktorsystem zurückgeführt wird.

**19.** Verfahren nach einem der vorhergehenden Ansprüche, bei dem weiter ein Gemisch, das Essigsäureanhydrid und Methylacetat enthält, von dem flüssigen Produktgemisch aus dem zweiten Flüssigphasenreaktor abgetrennt wird und mindestens ein Teil des Gemisches in den zweiten Flüssigphasenreaktor zurückgeführt wird.

**20.** Verfahren nach einem der vorhergehenden Ansprüche, bei dem das nicht umgesetzte Synthesegas aus dem ersten Flüssigphasenreaktor weiter Kohlendioxid enthält und wobei weiter mindestens ein Teil des Kohlendioxids in dem nicht umgesetzten Synthesegas vor dem zweiten Flüssigphasenreaktor entfernt wird.

**21.** Verfahren nach einem der vorhergehenden Ansprüche, bei dem vor dem zweiten Flüssigphasenreaktor mindestens ein Teil des Wasserstoffes von dem nicht umgesetzten Synthesegas aus dem ersten Flüssigphasenreaktor entfernt wird, wodurch die Synthese von Essigsäureanhydrid und Methylacetat gesteigert und die Synthese von Ethylidendiacetat und Essigsäure verringert wird.

## Revendications

1. Procédé de synthèse de composés acétylés oxygénés à partir de gaz de synthèse comprenant de l'hydrogène et du monoxyde de carbone, ledit procédé comprenant:

   la) la réaction dudit gaz de synthèse dans un premier réacteur en phase liquide en présence d'un catalyseur de synthèse du méthanol et d'un catalyseur de déshydratation du méthanol en suspension dans un liquide inerte pour produire du diméthyléther et du méthanol;
   (b) le retrait dudit premier réacteur d'un courant de produits intermédiaire comprenant du diméthyléther, du méthanol, de l'eau et du gaz de synthèse qui n'a pas réagi;
   (c) le passage dudit courant intermédiaire dans un second réacteur en phase liquide et la réaction dudit diméthyléther, dudit méthanol et dudit gaz de synthèse qui n'a pas réagi avec de l'acide acétique en présence d'un système catalytique consistant essentiellement en un métal du groupe VIII, en iodure de méthyle, en iodure de lithium et, éventuellement, en acétate de lithium pour produire un ou plusieurs composés acétylés oxygénés choisis parmi le diacétate d'éthylidène, l'acide acétique, l'anhydride acétique et l'acétate de méthyle; et
   (d) le retrait dudit second réacteur en phase liquide d'un mélange de produits liquides comprenant ledit ou lesdits composés acétylés oxygénés et un courant de vapeurs comprenant le gaz de synthèse qui n'a pas réagi.

2. Procédé selon la revendication 1 dans lequel ledit gaz de synthèse est produit par l'oxydation partielle d'une charge carbonée dans un système à réacteur d'oxydation partielle.

3. Procédé selon la revendication 2 dans lequel ladite charge carbonée est du gaz naturel.

4. Procédé selon l'une quelconque des revendications précédentes dans lequel ledit catalyseur de synthèse du méthanol est un catalyseur de $Cu/ZnO/Al_2O_3$.

5. Procédé selon l'une quelconque des revendications précédentes dans lequel ledit catalyseur de déshydratation du méthanol est de l'alumine obtenue par traitement thermique de la boehmite.

6. Procédé selon l'une quelconque des revendications précédentes dans lequel ledit catalyseur de synthèse du méthanol comprend du cuivre et ledit catalyseur de déshydratation du méthanol comprend de l'alumine, le catalyseur de synthèse du méthanol représentant entre 75 et 90% du catalyseur de synthèse du méthanol plus le catalyseur de déshydratation du méthanol sur une base massique.

7. Procédé selon l'une quelconque des revendications précédentes dans lequel ledit premier réacteur en phase liquide est exploité à une température comprise entre 225 et 270°C (440 et 520°F), à une pression comprise entre 5 et 14 MPa (750 et 2000 psig) et à un volume gazeux par masse par heure compris entre 3000 et 15000 litres standard/(kg de catalyseur-h).

8. Procédé selon l'une quelconque des revendications précédentes qui comprend en outre la séparation du méthanol et de l'eau dudit courant de produits intermédiaire par distillation et le recyclage d'au moins une partie du méthanol séparé dans ledit premier réacteur en phase liquide.

9. Procédé selon l'une quelconque des revendications précédentes dans lequel ledit métal du groupe VIII est le chlorure de rhodium (III) trihydraté.

10. Procédé selon l'une quelconque des revendications précédentes dans lequel ledit système catalytique comprend en outre de l'acétate de lithium.

11. Procédé selon l'une quelconque des revendications précédentes dans lequel le liquide dans ledit second réacteur en phase liquide comprend un ou plusieurs desdits composés acétylés oxygénés.

12. Procédé selon l'une quelconque des revendications précédentes dans lequel ledit second réacteur en phase liquide est exploité à une température comprise entre 120° et 195°C et à une pression comprise entre 3 et 15 MPa (400 et 2100 psig).

13. Procédé selon l'une quelconque des revendications précédentes dans lequel le diacétate d'éthylidène et l'acide acétique sont séparés par distillation dudit mélange de produits liquides.

14. Procédé selon la revendication 13 qui comprend en outre le recyclage d'une première partie dudit acide acétique dans ledit second réacteur en phase liquide.

15. Procédé selon la revendication 14 dans lequel ledit gaz de synthèse est produit par l'oxydation partielle d'une charge carbonée dans un système à réacteur d'oxydation partielle et dans lequel une seconde partie dudit acide acétique est recyclée dans ledit système à réacteur d'oxydation partielle.

16. Procédé selon la revendication 15 qui comprend en outre le recyclage d'au moins une partie du gaz de synthèse qui n'a pas réagi dudit second réacteur en phase liquide dans ledit système à réacteur d'oxydation partielle.

17. Procédé selon l'une quelconque des revendications 13 à 16 qui comprend en outre le passage dudit diacétate d'éthylidène dans un système à réacteur de pyrolyse dans lequel ledit diacétate d'éthylidène est converti en un produit intermédiaire comprenant de l'acétate de vinyle et de l'acide acétique, et la séparation dudit produit intermédiaire par distillation en acétate de vinyle et en acide acétique.

18. Procédé selon la revendication 17 dans lequel ledit gaz de synthèse est produit par l'oxydation partielle d'une charge carbonée dans un système à réacteur d'oxydation partielle et au moins une partie dudit acide acétique séparé dudit produit intermédiaire est recyclée dans ledit système à réacteur d'oxydation partielle.

19. Procédé selon l'une quelconque des revendications précédentes qui comprend en outre la séparation d'un mélange comprenant de l'anhydride acétique et de l'acétate de méthyle du mélange de produits liquides provenant dudit second réacteur en phase liquide et le recyclage d'au moins une partie dudit mélange dans ledit second réacteur en phase liquide.

20. Procédé selon l'une quelconque des revendications précédentes dans lequel ledit gaz de synthèse qui n'a pas réagi provenant dudit premier réacteur en phase liquide comprend en outre du dioxyde de carbone et en outre dans lequel au moins une partie du dioxyde de carbone dans ledit gaz de synthèse qui n'a pas réagi est retirée avant ledit second réacteur en phase liquide.

21. Procédé selon l'une quelconque des revendications précédentes dans lequel au moins une partie de l'hydrogène est retirée dudit gaz de synthèse qui n'a pas réagi provenant dudit premier réacteur en phase liquide avant ledit second réacteur en phase liquide ce qui augmente la production d'anhydride acétique et d'acétate de méthyle et réduit la production de diacétate d'éthylidène et d'acide acétique.

FIG. I

EP 0 566 370 B1

FIG.2

FIG.3

EP 0 566 370 B1